# FASCICULE DE BREVET EUROPEEN

(11) **EP 3 455 202 B1**
(45) Date de publication et mention de la délivrance du brevet: **17.02.2021**
(21) Numéro de dépôt: 17725551.0
(22) Date de dépôt: 11.05.2017
(51) Int. Cl.: C07C 51/44, C07C 59/08

(54) **PROCEDE DE PURIFICATION D'UNE SOLUTION AQUEUSE D'ACIDE LACTIQUE**
VERFAHREN ZUR REINIGUNG EINER WÄSSRIGEN MILCHSÄURELÖSUNG
METHOD FOR PURIFYING AN AQUEOUS LACTIC ACID SOLUTION

(30) Priorité: 11.05.2016 BE 201605333
(43) Date de publication de la demande: 20.03.2019
(73) Titulaire: Futerro S.A., 7760 Escanaffles (BE)
(72) Inventeur: WILLOCQ, Jonathan, B-7760 Escanaffles (BE); COSZACH, Philippe, B-7760 Escanaffles (BE); BOGAERT, Jean-Christophe, B-7760 Escanaffles (BE)
(74) Mandataire: Pronovem
(86) Numéro de dépôt international: PCT/EP2017/061386
(87) Numéro de publication internationale: WO 2017/194700

(56) Documents cités:
- WO-A1-01/38283
- WO-A1-98/55442
- KONSTANTINOS N GILNOS ET AL: "Design of Sidestream Distillation Columns", INDUSTRIAL AND ENGINEERING CHEMISTRY PROCESS DESIGN AND DEVELOPMENT, EASTON, PA, US, vol. 24, no. 3, 1 juillet 1985 (1985-07-01), pages 822-828, XP001273142, ISSN: 0196-4305

## Description

### Objet de l'invention

La présente invention se rapporte à un procédé de purification d'une solution aqueuse d'acide lactique, obtenue à partir d'un milieu de fermentation ou de toute autre source préalablement débarrassée des substances solides et/ou de la biomasse éventuellement présentes ainsi que des substances ioniques.

### État de la technique

Plusieurs procédés industriels de polymérisation de l'acide lactique ont été développés ces dernières années avec plus ou moins de succès. Une des voies préférées pour la production de PLA étant la polymérisation par ouverture de cycle. Cependant, quelque soit la voie envisagée (ouverture de cycle ou polycondensation), elle nécessite un acide lactique de départ d'une très grande pureté et donc débarrassé des impuretés issues de la fermentation des sucres ou de phénomènes de dégradation pouvant apparaitre durant sa production.

Le brevet EP0986532 décrit un procédé de purification de l'acide lactique obtenu par fermentation comprenant un prétraitement sur colonnes ioniques, une double étape de concentration de la solution d'acide lactique avec élimination de la totalité de l'eau libre et une distillation de l'acide concentré. Cependant, bien que ce procédé génère un acide lactique de qualité pour une grande partie des applications du marché traditionnel, il ne permet pas une séparation optimale des impuretés, telles que les mono- ou di-acides organiques, les alcools, les aldéhydes, etc... lors des étapes de purification. En effet, certaines de ces impuretés ont des volatilités telles qu'elles se retrouvent dans l'acide lactique purifié. Or ces impuretés provoquent, lors des étapes de synthèse du lactide (cyclisation de l'acide lactique), une augmentation de la couleur lors de l'étape de polycondensation ou un « blocage » de la réaction de back biting. Par ailleurs, elles sont aussi de nature à pouvoir désactiver les catalyseurs de polymérisation ou cyclisation couramment utilisés.

Le brevet US1594843 décrit une technique de purification de l'acide lactique par distillation instantanée (flash) d'une solution aqueuse d'acide lactique. Aucune référence n'est faite quant aux impuretés volatiles susceptibles de perturber la polymérisation néanmoins il apparait évident à l'homme du métier qu'une distillation flash ne permet pas d'obtenir une sélectivité suffisante pour séparer les différents constituants volatiles d'une solution.

Le brevet EP1232137, quant à lui, décrit une technique de purification d'une solution aqueuse d'acide lactique à l'aide d'au moins deux étapes de distillation, la solution étant mise en phase vapeur lors de la première étape de distillation et véhiculée vers une colonne de distillation. Le fait de recourir à deux étapes successives de distillation implique à la fois d'importants coûts d'investissement mais également des coûts opératoires, principalement énergétique, très élevés. De plus, les temps de séjour plus élevés de part la présence de deux colonnes successives, entrainent des dégradations plus nombreuses impactant sur la pureté et la couleur de l'acide lactique final.

Il existe donc un besoin pour une méthode économiquement efficiente de purification de l'acide lactique permettant l'élimination aussi bien des impuretés non-volatiles que des impuretés volatiles de type acides ou alcools de manière à obtenir un acide lactique purifié d'un grade permettant sa polymérisation dans des conditions optimales pour la production d'acide polylactique.

### Éléments caractéristiques de l'invention

La présente invention se rapporte à un procédé de purification d'une solution aqueuse d'acide lactique, obtenue à partir d'un milieu de fermentation ou de toute autre source préalablement débarrassée des substances solides et/ou de la biomasse éventuellement présentes ainsi que des substances ioniques, caractérisé en ce qu'il comprend les deux étapes suivantes :
a. Une concentration de la solution d'acide lactique jusqu'à atteindre une concentration comprise entre 85 et 95%, préférentiellement entre 90 et 95%, soit 15 à 5 % d'eau libre ou préférentiellement 10 à 5 % d'eau libre ;
b. Une distillation en colonne multi-étagée comportant trois zones et permettant la séparation en une seule étape de l'acide lactique, des composés volatils et des impuretés plus lourdes.

### Brève description des figures

La figure 1 montre schématiquement le procédé de purification de l'acide lactique selon une forme d'exécution de la présente invention.
La figure 2 montre schématiquement la deuxième étape (purification de l'acide lactique par distillation) du procédé selon une forme d'exécution de la présente invention.

### Description détaillée de l'invention

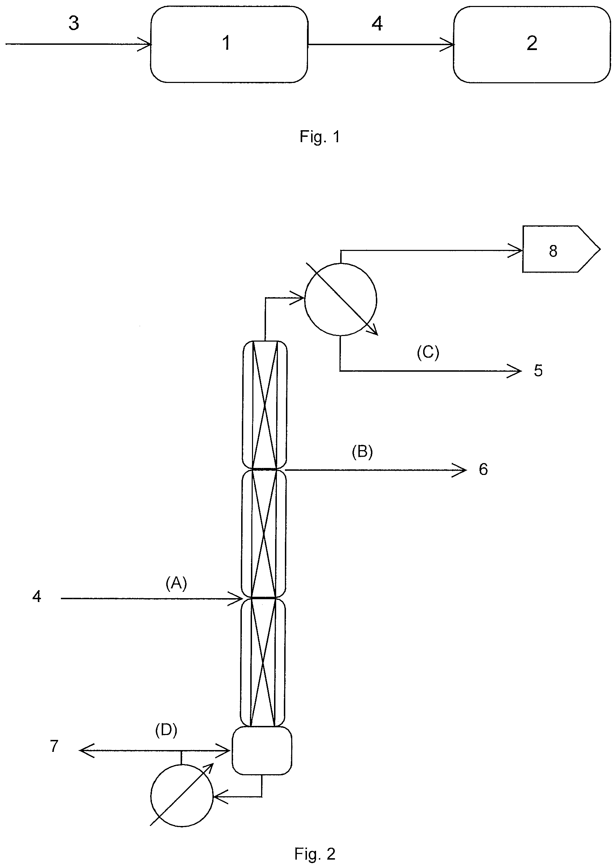

La présente invention décrit un procédé de purification de l'acide lactique provenant d'une solution aqueuse de cet acide telle qu'obtenue d'un milieu de fermentation ou de toute autre source préalablement débarrassée des substances solides et/ou de la biomasse éventuellement présentes ainsi que des substances ioniques par toute technique connue de l'homme de l'art comme par exemple les résines échangeuses ioniques, l'utilisation d'amines grasses quaternisées, la chromatographie,.... La figure 1 illustre le procédé de purification de l'acide lactique (AL) tel qu'il y est fait référence dans la présente invention. Ce procédé de l'invention comprend essentiellement les étapes suivantes:

### 1. Concentration de la solution d'acide lactique

Cette étape de l'invention consiste en la concentration rapide et à basse température de la solution d'acide lactique jusqu'à atteindre une concentration comprise entre 85 et 95%, préférentiellement entre 90 et 95%. Une approche préférée de la présente invention envisage la conduite de cette évaporation sous pression réduite, maintenue entre 40 et 500 mbar absolus et préférablement entre 50 et 250 mbar, afin d'assurer une température d'ébullition de la solution aussi basse que possible. Cette étape de l'invention est réalisée par toute technique connue de l'homme de l'art, telle que, par exemple, la couche mince et plus particulièrement l'évaporation en film ruisselant. Un mode préféré de cette étape de l'invention est d'utiliser une technique permettant un temps séjour minimal afin de réduire l'oligomérisation et ainsi améliorer le rendement global.

Selon un mode préféré de réalisation du procédé de l'invention, cette étape est directement suivie de l'étape de distillation, c'est-à-dire qu'il n'y a aucune période de stockage de la solution concentrée d'acide lactique entre les deux étapes du procédé. Cependant, selon un autre mode de réalisation du procédé de l'invention, on peut, afin de faciliter le transfert entre l'étape de concentration de la solution d'acide lactique et l'étape de distillation, prévoir une période stockage limitée de la solution concentrée d'acide lactique, c'est-à-dire une période comprise entre 0 et 24h, préférentiellement entre 0 et 120 min, plus préférentiellement entre 0 et 15 min, avant de la transférer à l'étape de distillation. Dans ce mode particulier de réalisation du procédé de l'invention et en fonction de la température de stockage, l'impact de cette période de stockage sera plus ou moins marqué (par ex. : un stockage même prolongé (48h voire plus) à une température inférieure à 20°C aura un impact moindre qu'un stockage de 2h à 140°C).

### 2. Purification de l'acide lactique par distillation

Cette étape se caractérise en ce que la solution concentrée d'acide lactique est soumise à une distillation en colonne multi-étagée comportant trois zones (figure 2) et contient un garnissage limitant au maximum le « hold up » (volume mort) et les pertes de charges, ce garnissage sera préférentiellement de type structuré. L'apport calorifique est assuré par un rebouilleur positionné en pied de colonne permettant de réduire au maximum toute dégradation thermique ou chimique, il s'agira préférentiellement d'un évaporateur de type couche mince à surface raclée ou non. La température de la paroi de l'évaporateur est maintenue entre 80 et 200°C, préférentiellement entre 100 et 180°C, plus préférentiellement entre 130 et 170°C. En tête de colonne se trouve un système de condensation ainsi qu'un système de reflux. L'acide lactique issu de l'étape 1 est alimenté dans la colonne au-dessus du rebouilleur (A). L'eau et les impuretés volatiles sont éliminées en tête de colonne (C) alors que l'acide lactique purifié est extrait sous forme liquide via une extraction latérale située entre l'alimentation et la tête de colonne (B). Enfin, les molécules les plus lourdes sortent en pied de colonne (D) et/ou bouclent sur le rebouilleur. L'utilisation d'une colonne unique permet de diminuer fortement les temps de résidence et donc les phénomènes de dégradation thermique et chimique, améliorant ainsi le rendement de la distillation mais également la pureté de l'acide lactique (en évitant la contamination).

La pression est comprise entre 10-3 et 100 mbar absolus, préférentiellement entre 10-1 et 20 mbar absolus, plus préférentiellement entre 1 et 10 mbar. L'unité est dûment opérée dans une gamme de températures comprise entre 80 et 200°C et une pression comprise entre 10-3 et 100 mbar absolus, préférentiellement dans une gamme de températures comprise entre 100 et 180°C et une pression comprise entre 10-1 et 20 mbar absolus. Plus préférentiellement, l'unité est opérée dans une gamme de températures comprise entre 130 et 170°C et une pression comprise entre 1 et 10 mbar absolus. Selon une variante améliorée, mais non essentielle de la présente invention, le résidu de purification peut être dirigé vers un second distillateur dans lequel les conditions de température et de pression sont plus drastiques. L'acide lactique issu de cette post-distillation et partiellement purifié peut être recyclé soit vers l'alimentation du distillateur principal, soit en amont du procédé.

Le résidu peut également être avantageusement recyclé, après hydrolyse en acide lactique, directement dans le flux d'entrée de la présente invention ou au niveau d'une des étapes de la pré-purification dans les procédés connus de production d'acide lactique (par exemple la chromatographie, les résines échangeuses d'ions, ...). Ce recyclage sera d'autant plus aisé que les impuretés volatiles responsables de la couleur ont été éliminées lors de l'étape de distillation.

D'autres détails et particularités de l'invention, donnés ci-après à titre d'exemples non-limitatifs, ressortent de la description comme quelques formes possibles de sa réalisation.

### Exemples

### Exemple 1

L'objectif de cet exemple est de prouver l'amélioration de la qualité de l'acide lactique par le procédé de l'invention par rapport à l'état de l'art.

Une solution d'acide lactique à 15% obtenue à partir d'un milieu de fermentation, débarrassée de la biomasse par filtration, de la couleur par passage sur charbon ainsi que des différentes substances ioniques par passage sur résine cationique et anionique, est séparée en deux flux : « témoin » et « invention ».

Dans une première expérience le flux « témoin » est concentré en deux étapes jusqu'à 100%, toute l'eau libre étant éliminée, conformément au brevet EP0986532. Il est alors alimenté sur un évaporateur couche-mince à surface raclée ayant une surface d'échange de 500 cm², surmonté d'un condenseur, afin de distiller l'acide lactique. Le fluide caloporteur circulant dans l'évaporateur est de l'huile thermique à 150°C, le vide appliqué sur l'installation est de 10 mbar absolu. L'acide lactique à distiller est alimenté à un débit de un litre par heure à l'aide d'une pompe péristaltique. L'acide lactique est récupéré avec un rendement de 56%.

Dans une seconde expérience, le flux « invention », est concentré en une seule étape à 95% (soit 5% d'eau résiduelle) dans un évaporateur à film ruisselant, il est ensuite distillé sur une colonne à garnissage structuré de type Sulzer EX. La colonne de distillation est décomposée en 3 zones : la zone allant du rebouilleur à l'alimentation (figure 2, A) comporte une hauteur de garnissage mesurant 5,5 cm, celle de l'alimentation à l'extraction (figure 2, B) comporte une hauteur de garnissage de 11 cm et celle de l'extraction à la tête de colonne une hauteur de garnissage de 11 cm également. L'acide lactique à 95% est alimenté au premier tiers de la colonne, tandis que l'acide lactique pur est soutiré, sous forme liquide, au deuxième tiers à l'aide d'une pompe péristaltique. Les composés les plus volatils quant à eux sont récupérés en tête de colonne à l'aide d'un condenseur équipé d'un système de reflux. Ce dernier est paramétré afin de refluer les distillats à raison de 50%. En pied de colonne, le rebouilleur consiste en un évaporateur couche-mince à surface raclée ayant une surface d'échange de 500 cm². Le fluide caloporteur circulant dans l'évaporateur est de l'huile thermique à 170°C, le vide appliqué sur l'installation est de 10 mbar absolu. L'acide lactique à distiller est alimenté à un débit de un litre par heure à l'aide d'une pompe péristaltique. Le rendement de distillation est de l'ordre de 53%.

Les résultats analytiques des acides lactiques distillés lors de ces deux premières expériences sont repris dans les tableaux 1 et 2.

**Tableau 1 : caractéristiques principales de l'acide lactique « témoin » et de l'acide lactique « invention » après distillation**

| | | **[Acide lactique]^{(a)} (%)** | **Fresh Color (Hazen)** | **Heat Stability (Hazen)** | **[Eau]^{(b)} (%)** |
|---|---|---|---|---|---|
| **Témoin** | Distillat | 99,67 | 17 | 94 | 1,0 |
| **Invention** | Produit d'intérêt (figure 2, B) | 99,89 | 12 | 23 | 0,7 |

| | | | | | |
|---|---|---|---|---|---|
| (a) Déterminé par titrage (b) Déterminé par Karl Fisher | | | | | |

**Tableau 2: analyse des impuretés organiques de l'acide lactique « témoin » et de l'acide lactique « invention » après distillation**

| | | **[5-HMF] ^{(c)} (ppm)** | **[2-F] ^{(c)} (ppm)** | **[2FMK] ^{(c)} (ppm)** | **[MFA] ^{(c)} (ppm)** | **[Acide pyruvique]^{(d)} (g/l)** | **[Acide propionique]^{(d)} (g/l)** |
|---|---|---|---|---|---|---|---|
| **Témoin** | Distillat | 41,28 | 15,69 | 0,27 | 1,09 | 0,46 | 2,58 |
| **Invention** | Produit d'intérêt (figure 2, B) | 0,73 | 2,17 | 6,06 | 1,03 | 0,17 | 2,41 |
| | | | | | | | |
| (a) Déterminé par HPLC UV | | | | | 5 HMF = 5-hydroxymethyl furfural | | |
| (b) Déterminé par HPLC acides organiques | | | | | 2-F = 2-Furfural | | |
| | | | | | 2-FMK = 2-furylmethylketone | | |
| | | | | | MFA = 5-methyl-2-furaldehyde | | |

Pour une même solution de départ, on remarque que l'acide lactique purifié obtenu par le procédé de l'invention présente une bien meilleure qualité que le résultat « témoin ». En effet, les paramètres de couleur (« Fresh Color » et « Heat-Stability ») sont bien plus bas pour l'acide lactique « invention ». On observe également une concentration près de 6 fois plus importante en molécules absorbant dans l'UV pour l'acide lactique « témoin » (58,33 ppm au total contre 9,99 ppm pour l'acide lactique purifié selon l'invention). L'impact sur la concentration en acide pyruvique est également significatif.

### Exemple 2

Dans ce deuxième exemple, la même solution d'acide lactique à 15% de l'exemple 1 est concentré en une seule étape à 90% (soit 10% d'eau résiduelle), il est ensuite distillé sur une colonne à garnissage structuré de type Sulzer EX selon le principe de l'invention dans les mêmes conditions et sur le même appareillage que ceux décrits dans l'exemple 1. Le rendement de distillation est de l'ordre de 51%.

Les résultats analytiques de l'acide lactique distillé sont repris dans les tableaux 3 et 4.

**Tableau 3 : caractéristiques principales de l'acide lactique et du distillat obtenu après traitement**

| | | **[Acide lactique]^{(a)} (%)** | **Fresh Color (Hazen)** | **Heat Stability (Hazen)** | **[Eau]^{(b)} (%)** |
|---|---|---|---|---|---|
| **Invention** | Produit d'intérêt (figure 2, B) | 99,73 | 11 | 18 | 0,8 |

| | | | | | |
|---|---|---|---|---|---|
| (a) Déterminé par titrage (b) Déterminé par Karl Fisher | | | | | |

**Tableau 4: analyse des impuretés organiques de l'acide lactique obtenu après traitement**

| | | **[5-HMF] ^{(c)} (ppm)** | **[2-F] ^{(c)} (ppm)** | **[2FMK] ^{(c)} (ppm)** | **[MFA] ^{(c)} (ppm)** | **[Acide pyruvique]^{(d)}** (g/l) | **[Acide propionique]^{(d )} (g/l)** |
|---|---|---|---|---|---|---|---|
| **Invention** | Produit d'intérêt (figure 2, B) | 0,69 | 2,34 | 7,09 | 0,89 | 0,18 | 2,02 |
| | | | | | | | |
| (a) Déterminé par HPLC UV | | | | | 5 HMF = 5-hydroxymethyl furfural | | |
| (b) Déterminé par HPLC acides organiques | | | | | 2-F = 2-Furfural | | |
| | | | | | 2-FMK = 2-furylmethylketone | | |
| | | | | | MFA = 5-methyl-2-furaldehyde | | |

On remarque que l'acide lactique obtenu par le procédé de l'invention présente toujours d'aussi bons résultats que dans l'exemple 1. En effet, les paramètres de couleur (« Fresh Color » et « Heat-Stability ») sont du même ordre, et donc bien meilleurs que le produit obtenu par l'expérience « témoin » et ce même avec une concentration résiduelle d'eau de 10%. L'impact sur les molécules absorbant dans l'UV est également confirmé.

### Exemple 3

Les acides lactiques « témoin » et « invention » purifiés de l'exemple 1, sont cyclisés et purifiés suivant le procédé décrit dans le brevet EP2222658.

Le lactide obtenu est ensuite polymérisé selon le procédé décrit dans le brevet BE1019059.

Dans cet exemple, « l'indice de jaune » (Yellow Index) permet de caractériser la pureté du PLA après polymérisation. Il s'agit d'un nombre calculé à partir de données spectrophotométriques, qui décrit le changement de couleur d'un échantillon de l'incolore au jaune. Selon la méthode ASTM, il y a une définition de la blancheur et du jaunissement. L'indice de jaunissement ASTM E-313-98 est utilisé pour déterminer dans quelle mesure la couleur d'un échantillon se déplace en dehors d'un blanc idéal. Dans le cas présent, « l'indice de jaune » a été mesuré sur un spectro-guide sphere gloss BYK S 6836. Les résultats analytiques du PLA « témoin » et du PLA « invention » sont repris dans le tableau 5.

**Tableau 5: analyse de la couleur du PLA**

| | Yellow Index |
|---|---|
| PLA « témoin » | 8 |
| PLA « invention » | 2 |

On remarque que le PLA « invention » a un Yellow Index inférieur à celui du PLA « témoin », il est donc moins coloré et se rapproche plus d'un état de transparence.

### Repères de référence

- 1: étape de concentration
- 2: étape de distillation
- 3: solution aqueuse de l'acide lactique (concentration AL 5-15%)
- 4: solution concentrée de l'acide lactique (concentration AL 85-95%)
- 5: l'eau et les impuretés volatiles
- 6: l'acide lactique purifié
- 7: résidu
- 8: vide

## Revendications

1. Procédé de purification d'une solution aqueuse d'acide lactique, obtenue à partir d'un milieu de fermentation ou de toute autre source préalablement débarrassée des substances solides et/ou de la biomasse éventuellement présentes ainsi que des substances ioniques, **caractérisé en ce qu'**il comprend les deux étapes suivantes :
a. Une concentration de la solution d'acide lactique jusqu'à atteindre une concentration comprise entre 85 et 95%, préférentiellement entre 90 et 95%, soit 15 à 5 % d'eau libre ou préférentiellement 10 à 5 % d'eau libre ;
b. Une séparation en une seule étape du flux concentré obtenu en (a) en un flux comportant l'acide lactique purifié, un flux comportant les impuretés volatiles et un flux comportant les impuretés plus lourdes.

2. Procédé de purification selon la revendication 1, **caractérisé en ce que** la concentration (a) est réalisée sur un évaporateur couche mince et préférentiellement sur un évaporateur à film ruisselant.

3. Procédé de purification selon les revendications 1 et 2, **caractérisé en ce que** l'étape de concentration (a) est effectuée à pression réduite, entre 40 et 500 mbar, préférentiellement entre 50 et 250 mbar.

4. Procédé de purification selon la revendication 1, **caractérisé en ce que** l'étape (b) de séparation est opérée dans une colonne de distillation, préférentiellement dans une colonne de rectification.

5. Procédé de purification selon les revendications 1 et 4, **caractérisé en ce que** le flux comportant l'acide lactique purifié obtenu en (b) est extrait sous forme liquide via une extraction latérale.

6. Procédé de purification selon la revendication 4, **caractérisé en ce que** la colonne de distillation contient un garnissage structuré ou non limitant au maximum le temps de séjour et les pertes de charge.

7. Procédé de purification selon les revendications 1 à 6, **caractérisé en ce que** l'apport calorifique de la colonne de distillation est assuré par un rebouilleur permettant de réduire au maximum toute dégradation thermique et chimique.

8. Procédé de purification selon la revendication 7, **caractérisé en ce que** le rebouilleur sera préférentiellement un évaporateur de type couche mince à surface raclée ou non.

9. Procédé de purification selon la revendication 8, **caractérisé en ce que** la température au niveau du rebouilleur est comprise entre 80 et 200°C, préférentiellement entre 100 et 180°C et plus préférentiellement entre 130 et 170°C.

10. Procédé de purification selon les revendications 4 à 7, **caractérisé en ce que** la distillation est réalisée à une pression comprise entre 10⁻³ et 100 mbar absolus, préférentiellement entre 10⁻¹ et 20 mbar absolus et plus préférentiellement entre 1 et 10 mbar absolus.

11. Procédé de purification selon la revendication 1, **caractérisé en ce que** les étapes (a) et (b) du procédé sont séparées par une période de stockage comprise entre 0 et 24h, préférentiellement entre 0 et 120 min, plus préférentiellement entre 0 et 15 min.

12. Procédé de purification d'une solution aqueuse d'acide lactique, obtenue à partir d'un milieu de fermentation ou de toute autre source préalablement débarrassée des substances solides et/ou de la biomasse éventuellement présentes ainsi que des substances ioniques, **caractérisé en ce qu'**il consiste à :
a. Concentrer la solution d'acide lactique jusqu'à atteindre une concentration comprise entre 85 et 95%, préférentiellement entre 90 et 95%, soit 15 à 5 % d'eau libre ou préférentiellement 10 à 5 % d'eau libre ;
b. Stocker la solution concentrée obtenue en (a) pendant une période ne dépassant pas 24h ;
c. Séparer en une seule étape le flux concentré obtenu en (a) et stocké en (b) en un flux comportant l'acide lactique purifié, un flux comportant les impuretés volatiles et un flux comportant les impuretés plus lourdes.

13. Procédé de purification selon la revendication 12, **caractérisé en ce que** l'étape (c) de séparation est opérée dans une colonne de distillation, préférentiellement dans une colonne de rectification.

14. Procédé de purification selon l'une quelconque des revendications 1 et 13, **caractérisé en ce que** la solution d'acide lactique issue de l'étape (a) de concentration est alimentée dans la colonne, au-dessus du rebouilleur (A), l'acide lactique purifié est extrait, sous forme liquide, latéralement entre l'alimentation et la tête de colonne (B) tandis que l'eau et les impuretés volatiles sont éliminées en tête de colonne (C). Les molécules les plus lourdes sortent en pied de colonne (D) et/ou bouclent sur le rebouilleur.

15. Procédé de purification selon l'une quelconque des revendications précédentes, **caractérisé en ce que** le résidu de distillation peut être soit hydrolysé soit dirigé vers un second distillateur afin d'être recyclé soit vers l'alimentation du distillateur principal, soit en amont du procédé.

## Patentansprüche

1. Verfahren zur Reinigung einer wässrigen Milchsäurelösung, erhalten ausgehend von einem Fermentationsmedium oder jeder anderen Quelle, die zuvor von den festen Substanzen und/oder der Biomasse befreit wurde, die eventuell vorhanden sind, ebenso wie von den ionischen Substanzen, **dadurch gekennzeichnet, dass** es die zwei folgenden Schritte umfasst:
a. ein Konzentrieren der Milchsäurelösung, bis eine Konzentration im Bereich zwischen 85 und 95 %, vorzugsweise zwischen 90 und 95 % erhalten wird, d. h. mit 15 bis 5 % freiem Wasser oder vorzugsweise 10 bis 5 % freiem Wasser;
b. ein Trennen in einem einzigen Schritt des konzentrierten Flusses, erhalten in (a) in einen Fluss, umfassend die gereinigte Milchsäure, einen Fluss, umfassend die flüchtigen Verunreinigungen und einen Fluss, umfassend die schwereren Verunreinigungen.

2. Verfahren zur Reinigung nach Anspruch 1, **dadurch gekennzeichnet, dass** die Konzentration (a) auf einem Dünnschichtverdampfer und vorzugsweise auf einem Rieselfilmverdampfer durchgeführt wird.

3. Verfahren zur Reinigung nach Anspruch 1 und 2, **dadurch gekennzeichnet, dass** der Schritt des Konzentrierens (a) mit reduziertem Druck zwischen 40 und 500 mbar, vorzugsweise zwischen 50 und 250 mbar durchgeführt wird.

4. Verfahren zur Reinigung nach Anspruch 1, **dadurch gekennzeichnet, dass** der Schritt (b) des Trennens in einer Destillationssäule, vorzugsweise in einer Rektifiziersäule erfolgt.

5. Verfahren zur Reinigung nach Anspruch 1 und 4, **dadurch gekennzeichnet, dass** der Fluss, umfassend die gereinigte Milchsäure, erhalten in (b), in flüssiger Form mit Hilfe einer seitlichen Extraktion extrahiert wird.

6. Verfahren zur Reinigung nach Anspruch 4, **dadurch gekennzeichnet, dass** die Destillationssäule eine strukturierte oder nicht, auf die maximale Anwesenheitsdauer und die Ladeverluste begrenzte Auskleidung enthält.

7. Verfahren zur Reinigung nach Anspruch 1 bis 6, **dadurch gekennzeichnet, dass** der Wärmeeintrag der Destillationssäule durch einen Reboiler sichergestellt wird, der ermöglicht, jeden thermischen und chemischen Abbau maximal zu reduzieren.

8. Verfahren zur Reinigung nach Anspruch 7, **dadurch gekennzeichnet, dass** der Reboiler vorzugsweise ein Verdampfer vom Dünnschicht-Typ mit oder ohne abgeschabter Oberfläche ist.

9. Verfahren zur Reinigung nach Anspruch 8, **dadurch gekennzeichnet, dass** die Temperatur auf der Ebene des Reboilers im Bereich zwischen 80 und 200 °C, vorzugsweise zwischen 100 und 180 °C, und noch bevorzugter zwischen 130 und 170 °C liegt.

10. Verfahren zur Reinigung nach Anspruch 4 bis 7, **dadurch gekennzeichnet, dass** die Destillation mit einem Druck durchgeführt wird, der im Bereich zwischen 10⁻³ und 100 mbar absolut, vorzugsweise zwischen 10⁻¹ und 20 mbar absolut und noch bevorzugter zwischen 1 und 10 mbar absolut liegt.

11. Verfahren zur Reinigung nach Anspruch 1, **dadurch gekennzeichnet, dass** die Schritte (a) und (b) des Verfahrens durch einen Speicherzeitraum getrennt sind, der im Bereich zwischen 0 und 24h, vorzugsweise zwischen 0 und 120 min, noch bevorzugter zwischen 0 und 15 min liegt.

12. Verfahren zur Reinigung einer wässrigen Milchsäurelösung, erhalten ausgehend von einem Fermentationsmedium oder jeder anderen Quelle, die zuvor von den festen Substanzen und/oder der Biomasse befreit wurde, die eventuell vorhanden sind, ebenso wie von den ionischen Substanzen, **dadurch gekennzeichnet, dass** es aus Folgendem besteht:
a. Konzentrieren der Milchsäurelösung, bis eine Konzentration im Bereich zwischen 85 und 95 %, vorzugsweise zwischen 90 und 95% erhalten wird, d. h. 15 bis 5 % freiem Wasser oder vorzugsweise 10 bis 5 % freiem Wasser;
b. Speichern der konzentrierten Lösung, erhalten in (a), während eines Zeitraums, der 24 h nicht übersteigt,
c. Trennen in einem einzigen Schritt des konzentrierten Flusses, erhalten in (a) und gelagert in (b) in einen Fluss, umfassend die gereinigte Milchsäure, einen Fluss, umfassend die flüchtigen Verunreinigungen und einen Fluss, umfassend die schwereren Verunreinigungen.

13. Verfahren zur Reinigung nach Anspruch 12, **dadurch gekennzeichnet, dass** der Schritt (c) des Trennens in einer Destillationssäule, vorzugsweise in einer Rektifiziersäule erfolgt.

14. Verfahren zur Reinigung nach irgendeinem der Ansprüche1 bis 13, **dadurch gekennzeichnet, dass** die Milchsäurelösung, stammend aus Schritt (a) des Konzentrierens, in der Säule, über dem Reboiler (A) versorgt wird, die gereinigte Milchsäure in flüssiger Form seitlich zwischen der Versorgung und dem Säulenkopf (B) extrahiert wird, während das Wasser und die flüchtigen Verunreinigungen am Säulenkopf (C) eliminiert werden. Die schwersten Moleküle treten am Säulenfuß (D) aus und/oder umgeben den Reboiler.

15. Verfahren zur Reinigung nach irgendeinem der vorhergehenden Ansprüche, **dadurch gekennzeichnet, dass** der Destillationsrückstand entweder hydrolysiert oder an ein zweites Destilliergerät geschickt werden kann, um entweder zur Versorgung des Hauptdestilliergeräts oder vorgelagert vom Verfahren recycelt zu werden.

## Claims

1. Method for purifying an aqueous lactic acid solution obtained from a fermentation medium or any other source previously stripped of solid substances and/or biomass which may be contained therein as well as ionic substances, **characterized in that** it comprises the two following steps:
a. concentrating the lactic acid solution until it reaches a concentration of between 85 and 95 %, preferably between 90 and 95 %, i.e. 15 to 5 % free water or preferably 10 to 5 % free water;
b. separating, in one single step, the concentrated stream obtained at (a) into a stream comprising the purified lactic acid, a stream comprising the volatile impurities and a stream comprising the heavier impurities.

2. Purification method according to claim 1, **characterized in that** concentration (a) is performed on a thin film evaporator and preferably on a falling film evaporator.

3. Purification method according to claims 1 and 2, **characterized in that** the concentration step (a) is performed under reduced pressure, between 40 and 500 mbar, preferably between 50 and 250 mbar.

4. Purification method according to claim 1, **characterized in that** the separation step (b) is carried out in a distillation column, preferably in a rectifying column.

5. Purification method according to claims 1 and 4, **characterized in that** the stream containing the purified lactic acid obtained at (b) is extracted in liquid form via side-stream extraction.

6. Purification method according to claim 4, **characterized in that** the distillation column has a structured or not lining, limiting to a maximum the residence time and head losses.

7. Purification method according to claims 1 to 6, **characterized in that** the heat input of the distillation column is ensured by a reboiler allowing a maximum reduction in any thermal and chemical degradation.

8. Purification method according to claim 7, **characterized in that** the reboiler is preferably an thin film type evaporator with or without scraped surface.

9. Purification method according to claim 8, **characterized in that** the temperature at the reboiler is between 80 and 200 °C, preferably between 100 and 180 °C and more preferably between 130 and 170 °C.

10. Purification method according to claims 4 to 7, **characterized in that** the distillation is performed at a pressure of between 10⁻³ and 100 mbar absolute, preferably between 10⁻¹ and 20 mbar absolute, and more preferably between 1 and 10 mbar absolute.

11. Purification method according to claim 1, **characterized in that** steps (a) and (b) of the method are separated by a storage period of between 0 and 24 h, preferably between 0 and 120 min, more preferably between 0 and 15 min.

12. Method for purifying an aqueous lactic acid solution obtained from a fermentation medium or any other source previously stripped of solid substances and/or biomass which may be contained therein as well as ionic substances, **characterized in that** it consists of:
a. Concentrating the lactic acid solution until it reaches a concentration of between 85 and 95 %, preferably between 90 and 95 %, i.e. 15 to 5 % free water or preferably 10 to 5 % free water;
b. Storing the concentrated solution obtained at (a) for a period not exceeding 24 h;
c. Separating, in one single step, the concentrated stream obtained at (a) and stored at (b) into a stream comprising the purified lactic acid, a stream comprising the volatile impurities and a stream comprising the heavier impurities.

13. Purification method according to claim 12, **characterized in that** the separation step (c) is carried out in a distillation column, preferably in a rectifying column.

14. Purification method according to any of claims 1 and 13, **characterized in that** the lactic acid solution derived from concentration step (a) is fed into the column above the reboiler (A), the purified lactic acid is laterally extracted in liquid form between the feed and the head of the column (B), whilst the water and the volatile impurities are removed at the head of the column (C). The heaviest molecules leave via the foot of the column (D) and/or are looped on the reboiler.

15. Purification method according to any of the preceding claims, **characterized in that** the distillation residue can be either hydrolysed or directed towards a second distiller for its recycling either towards the feed of the main distiller, or upstream of the method.
